# EUROPEAN PATENT APPLICATION

(11) **EP 2 255 876 A2**
(43) Date of publication of application: **01.12.2010**
(21) Application number: 10169789.4
(22) Date of filing: 27.10.2003
(51) Int. Cl.: B01J 23/58, C07D 301/10

(54) **A process for preparing an olefin oxide, a method of using the olefin oxide and a catalyst composition**

(30) Priority: 28.10.2002 US 421752 P
(62) Divisional of application: 03776573.2
(71) Applicant: Shell Internationale Research Maatschappij B.V., 2596 HR The Hague (NL)
(72) Inventor: Rubinstein, Leonid Isaakovich, Houston, TX 77006 (US); Gutierrez, Candido, Houston, TX 77082 (US)
(74) Representative: Matthezing, Robert Maarten

(57) **Abstract**

The present invention provides a process for preparing an olefin oxide by reacting an olefin having at least three carbon atoms with oxygen in the presence of a catalyst composition containing silver and an alkali metal promoter deposited on a carrier, which alkali metal promoter contains potassium in a quantity of at least 5 µmole/g, relative to the weight of the catalyst composition, and a selectivity and work rate enhancing amount of an alkali metal selected from the group consisting of lithium and sodium and mixtures thereof. The invention also relates to a method for making a 1,2-diol or a 1,2-diol ether using the olefin oxide so prepared. Additionally, the invention relates to a catalyst composition comprising silver and a promoter deposited on a carrier.

## Description

### Field of the Invention

This invention relates to a process for preparing an olefin oxide by reacting an olefin having at least three carbon atoms with oxygen in the presence of a catalyst composition comprising silver and a promoter deposited on a carrier. The invention also relates to a method of using the olefin oxide so prepared for making a 1,2-diol or a 1,2-diol ether. Additionally, the invention relates to a catalyst composition comprising silver and a promoter deposited on a carrier.

### Background of the Invention

Olefins can be oxidized to the corresponding olefin oxide by direct oxidation, using molecular oxygen as the oxidant. The catalysts used in this oxidation comprise silver as a catalytically active metal deposited on a carrier. Most such catalysts contain a porous, inert support or carrier such as α-alumina upon which the silver and promoters are deposited.

In olefin oxidation, catalyst performance may be assessed on the basis of selectivity, activity and stability of operation. The selectivity is the percentage of the olefin in the feed stream yielding the desired olefin oxide. As the catalyst ages, the percentage of the olefin reacted normally decreases with time and to maintain a constant level of olefin oxide production, the temperature of the reaction is increased. However, this adversely affects the selectivity of the conversion to the desired olefin oxide. Because the reactor equipment can withstand temperatures only up to a certain level, it is necessary to terminate the reaction when the temperature reaches an unacceptable level. Thus, the longer the selectivity can be maintained at a high level and the oxidation can be performed at an acceptable temperature, the longer the catalyst charge can be kept in the reactor and the more product is obtained. Quite modest improvements in the maintenance of selectivity over long periods potentially yield large dividends in terms of process efficiency.

Many studies have been carried out to improve and optimize catalyst performance in the oxidation of ethylene in commercial ethylene oxide manufacturing plants. However, so far, no commercially feasible process has been found for a similar, direct oxidation of most higher olefins, particularly propylene.

US-A-3962136 teaches the use of catalyst compositions for the oxidation of ethylene to ethylene oxide which catalyst compositions consist essentially of silver and defined quantities of an alkali metal deposited on a refractory support.

US-A-4833261 teaches a process for the production of ethylene oxide by contacting ethylene with an oxygen containing gas in the presence of a catalyst composition comprising silver, a promoter of an alkali metal and a promoter of rhenium supported on a refractory support. The alkali metal is preferably potassium, rubidium or cesium or mixtures thereof. A long list of combinations of alkali metals is given.

US-A-4168247 teaches to employ in the oxidation of olefins a catalyst which comprises a promoting amount of sodium together with at least one of potassium, rubidium or cesium.

US-A-5625084 and US-A-5770746 teach the direct oxidation of propylene to propylene oxide in the presence of a catalyst comprising silver deposited on an alkaline earth metal carbonate, and comprising a potassium salt of a potassium cation and a nitrogen oxyanion or precursor thereof. In addition, the catalyst may comprise a promoting amount of a molybdenum promoter. In these documents there is no teaching as regards alkali metals other than potassium.

US-A-5698719 teaches the oxidation of propylene using a catalyst comprising silver deposited on calcium carbonate, and further comprising potassium nitrate.

US-A-5387751 discloses the direct oxidation of an olefin, for example ethylene and propylene, in the presence of a silver containing catalyst and in the presence of a nitrate or nitrite forming substance. In a long list of preferred embodiments elements are specified which may be present in the catalyst. The list comprises alkali metals, alkaline earth metals and transition metals.

According to US-A-5770746 and US-A-5625084, it is known that the catalysts and the reaction conditions which are best suited for ethylene oxide production do not give comparable results in the direct oxidation of higher olefins such as propylene. According to US-A-5698719, a problem with the catalytic vapor phase oxidation of propylene with molecular oxygen, as compared with ethylene oxidation, has been the generally poor selectivity attainable at a reasonable level of conversion.

Therefore, it is known that the catalysts and reaction conditions which are best suited for ethylene oxide production often do not give comparable results in the direct oxidation of higher olefins such as propylene. The discovery of processes capable of providing propylene oxide by vapor phase direct oxidation in higher yields and selectivities than are presently attainable thus would be most desirable.

The olefin oxides are important starting materials for the production of 1,2-diols or 1,2-diol ethers. In many cases the olefin oxides have properties which make them less suitable for transportation over long distances. For this reason, transportation of the olefin oxides is frequently avoided by converting the olefin oxides directly after their production into the corresponding 1,2-diol or a 1,2-diol ethers, which are better suited for transportation.

### Summary of the Invention

The present invention provides a catalyst composition comprising silver, an alkali metal promoter and a carrier, which alkali metal promoter comprises potassium in a quantity of at least 5 µmole/g, relative to the weight of the catalyst composition, and at least 1 µmole/g of an alkali metal selected from the group consisting of lithium and sodium and mixtures thereof.

The present invention further provides a process for preparing an olefin oxide which process comprises reacting an olefin having at least three carbon atoms with oxygen and in the presence of a catalyst composition comprising silver, an alkali metal promoter and a carrier which alkali metal promoter comprises potassium in a quantity of at least 5 µmole/g, relative to the weight of the catalyst composition, and at least 1 µmole/g of an alkali metal selected from the group consisting of lithium and sodium and mixtures thereof.

Additionally, a method is provided for producing a 1,2-diol or a 1,2-diol ether which method comprises converting the olefin oxide into the 1,2-diol or the 1,2-diol ether wherein the olefin oxide has been obtained by a process as described above.

### Detailed Description of the Invention

In accordance with the invention, in the oxidation of higher olefins with oxygen an improved catalyst performance can be achieved by employing a supported silver catalyst which further comprises a certain combination of alkali metal promoters. This is in particular the case when the oxidation is carried out in the additional presence of a nitrate or nitrite forming substance. By the term "improved catalyst performance" it is meant that there is an improvement in at least one of the catalyst properties, which catalyst properties include catalyst activity, selectivity, activity or selectivity performance over time, operability (i.e. resistance to run-away), conversion and work rate. Therefore, the present invention provides a process for preparing an olefin oxide which process comprises reacting an olefin having at least three carbon atoms with oxygen and in the presence of a catalyst composition comprising silver, an alkali metal promoter and a carrier, which alkali metal promoter comprises potassium and in addition lithium or sodium.

The present invention also provides a method for making a 1,2-diol or a 1,2-diol ether comprising converting an olefin oxide into the corresponding 1,2-diol or 1,2-diol ether wherein the olefin oxide has been obtained by a process according to this invention.

The carrier material for the catalyst may be of any kind of material suitable for supporting a catalyst and having the necessary physical and chemical properties to withstand a chemical process such as oxidation. For example, carriers may be selected from materials based on charcoal, magnesia, zirconia, Fuller's earth, kieselguhr, and artificial and natural zeolites. Preferred carriers comprise an alkaline earth metal carbonate, in particular, magnesium carbonate and more in particular, calcium carbonate. Other preferred carriers are alumina-, silica- or titania-based compounds and combinations thereof, such as alumina-silica based compounds, in particular alpha-alumina based compounds.

Typically, the carrier is a porous carrier, preferably having a specific surface area of from 0.01 m²/g to 50 m²/g, more preferably 0.03 m²/g to 40 m²/g, and most preferably from 0.05 m²/g to 30 m²/g, as measured by the B.E.T. method, and an apparent porosity of from 0.05 ml/g to 3 ml/g, preferably 0.07 ml/g to 2.5 ml/g, and more preferably from 0.1 ml/g to 2 ml/g, as measured by conventional water absorption technique. The B.E.T. method as referred to herein has been described in detail in S. Brunauer, P.Y. Emmett and E. Teller, J. Am. Chem. Soc. 60, 309-16 (1938).

Of particular interest are alpha-aluminas which have a specific surface area of from 0.1 m²/g to 25 m²/g, preferably 0.2 m²/g to 15 m²/g, and more preferably from 0.3 m²/g to 10 m²/g, as measured by the B.E.T. method, and which have an apparent porosity of from 0.1 ml/g to 0.6 ml/g, and, preferably from 0.1 ml/g to 0.55 ml/g, as measured by conventional water absorption technique. Preferably, these alpha-aluminas have a relatively uniform pore diameter. Specific examples of such alpha-aluminas are marketed by NorPro under the trademark ALUNDUM and by Südchemie.

Also of particular interest are alkaline earth metal carbonates, in particular calcium carbonate or magnesium carbonate, which have a specific surface area of from 1 m²/g to 20 m²/g, preferably 2 m²/g to 18 m²/g, and more preferably from 3 m²/g to 15 m²/g, as measured by the B.E.T. method, and which have an apparent porosity of from 0.05 ml/g to 2 ml/g, preferably 0.07 ml/g to 1.7 ml/g, and more preferably from 0.1 ml/g to 1.5 ml/g, as measured by conventional water absorption technique. The alkaline earth metal carbonate carriers are of particular interest as they provide catalysts which have an improved activity performance over time. The preferred alkaline earth metal carbonate carrier is one which has been bonded with silver. The silver bonded alkaline earth metal carbonate carrier is characterized by a high relative surface area, and a minimum compressive strength of 22N (5 lbs), and comprises 80-99% by weight alkaline earth metal carbonate and 1-20% by weight of silver, more preferably 85-97% by weight alkaline carbonate and 3-15% by weight silver and most preferably 90-95% by weight alkaline earth metal carbonate and 5-10% by weight silver. The silver bonded alkaline earth metal carbonate carrier may be made by mixing a commercially available alkaline earth metal carbonate powder with a silver oxalate ethylenediamine complex, having a concentration of silver from 15 to 33% by weight, preferably from 27-33%, in such quantities that the final ratio of silver/alkaline earth metal carbonate is approximately from 1:5 to 1:100, preferably from 1:6 to 1:30, more preferably from 1:8 to 1:10, and for example 1:9. After mixing the above components, an organic extrusion aide such as starch and optionally a burnout material may be added to the mixture, such that there are 90-100 parts by weight (pbw) calcium carbonate mixed with 1-2 pbw of the extrusion aid. Then, a sufficient amount of water, generally 35-45 pbw silver solution, may be added to make the composition extrudable, and the resulting composition may be mixed until homogeneous and extrudable. The resulting paste may then be extruded. One method of extrusion may be to force the paste through a die of from 0.5 mm to 5 cm, particularly from 1 mm to 5 mm. The extrudate may then be fired at a temperature ranging from 180 °C to 870 °C, particularly from 200 °C to 750 °C for 1-12 hours. The resulting extrudate may also first be dried over a period of 1 hour to 18 hours at for example from 10 °C to 500 °C, particularly from 50 °C to 200 °C, more particularly from 80 °C to 120 °C and then fired. An example of a program for firing the catalyst may be: an 0.1-10 hour ramp, such as 1 hour ramp, from 200 °C to 250 °C, held for 1 hour, then a 4 hour ramp from to 500 °C and held for 5 hours. The resulting catalyst carrier has good mechanical properties, particularly crush strength, and is suitable to manufacture the catalysts of the invention useful for oxidation of olefins.

Regardless of the carrier material used, it may be shaped into particles, chunks, pieces, and the like. Preferably, for use in a tubular fixed bed reactor, they are formed into a rounded shape, for example in the form of spheres, pellets, cylinders, rings or tablets, typically having dimensions in the range of from 2 mm to 2 cm. The term "shaped" is interchangeable with the term "formed".

The quantity of silver which may be supported on the carrier may be selected within wide ranges. Suitably, the quantity of silver is in the range of from 0.5 %w to 60 %w, preferably 0.7 %w to 58 %w, and more preferably from 1 %w to 55 %w, relative to the weight of the catalyst composition.

In accordance with this invention, the catalyst comprises, as promoters, alkali metals in the combination of potassium and sodium or lithium. The combination of potassium and lithium is preferred over the combination potassium and sodium. It is however more preferred to combine potassium with lithium and sodium. Further alkali metals may or may not be present. It has unexpectedly been found that the additional presence of rubidium or in particular cesium is advantageous. Eligible combinations are potassium, lithium, and rubidium; potassium, sodium, and rubidium; potassium, lithium, and cesium; potassium, sodium, and cesium; potassium, lithium, sodium, and rubidium; potassium, lithium, sodium, rubidium, and cesium; and in particular potassium, lithium, sodium, and cesium.

The quantity of potassium is typically at least 5 µmole/g, preferably at least 10 µmole/g, and it is typically at most 10 mmol/g or may be at most 1 mmol/g, relative to the weight of the catalyst composition. If the carrier is an alpha-alumina, it is preferred that the quantity of potassium is at least 5 µmole/g, preferably at least 10 µmole/g, and independently at most 0.5 mmol/g, preferably at most 0.2 mmol/g, on the same basis. If the carrier is an alkaline earth metal carbonate, typically calcium carbonate, it is preferred that the quantity of potassium is at least 10 µmole/g, in particular at least 50 µmole/g, and independently at most 10 mmol/g, in particular at most 5 mmol/g, on the same basis.

The total quantity of sodium and lithium is typically at least 1 µmole/g, and it is typically at most 10 mmol/g, relative to the weight of the catalyst composition. If the carrier is an alpha-alumina, it is preferred that the total quantity of sodium and lithium is at least 1 µmol/g, more preferably at least 5 µmol/g, and independently at most 0.5 mmol/g, more preferably at most 0.1 mmol/g, on the same basis. If the carrier is an alkaline earth metal carbonate, typically calcium or magnesium carbonate, it is preferred that the total quantity of sodium and lithium is at least 5 µmole/g, in particular at least 10 µmol/g, and independently at most 10 mmol/g, in particular at most 5 mmol/g, on the same basis.

If sodium and lithium containing promoters are both deposited on the carrier, the sodium/lithium molar ratio is typically in the range of from 0.01 to 100, more typically in the range of from 0.1 to 10.

The total quantity of rubidium and cesium is typically at least 0.01 µmole/g, and it is typically at most 1 mmol/g, relative to the weight of the catalyst composition. If the carrier is an alpha-alumina, it is preferred that the total quantity of rubidium and cesium is at least 0.01 µmol/g, in particular at least 0.1 µmol/g, and independently at most 0.1 mmol/g, in particular at most 0.05 mmol/g, on the same basis. If the carrier is an alkaline earth metal carbonate, typically calcium carbonate, it is preferred that the total quantity of rubidium and cesium is at least 0.1 µmole/g, in particular at least 1 µmole/g, and independently at most 1 mmol/g, in particular at most 0.2 mmol/g, on the same basis.

If rubidium and cesium containing promoters are both deposited on the carrier, the rubidium/cesium molar ratio is typically in the range of from 0.01 to 100, more typically in the range of from 0.1 to 10.

The skilled person will appreciate that the quantities of alkali metal promoters as defined are not necessarily the total amounts of these metals present in the catalyst composition. The quantities as defined are the quantities which have been added to the catalyst, e.g. by impregnation with suitable solutions of compounds of the alkali metals, such as salts or complexes of the alkali metals. These quantities do not include quantities of alkali metals which are locked into the carrier, for example by calcining, or are not extractable in a suitable solvent such as water or lower alcohol or amine or mixtures thereof and, therefore, do not provide a promoting activity. The skilled person will also appreciate that the carrier itself may be a source of the alkali metal promoter which may be used to impregnate the carrier. That is, the carrier may contain alkali metals which can be extracted with a suitable solvent, thus preparing an impregnating solution from which the alkali metal ions are deposited or re-deposited on the carrier.

The catalysts may be prepared in accordance with methods such as those known from US-A-3962136 and WO-00/15333.

In a suitable method of catalyst preparation, the carrier is impregnated with a liquid composition of compounds of silver, potassium, sodium and/or lithium and, if desirable with further compounds of, for example, rubidium and/or cesium, and subsequently dried by heating at a temperature of in the range of from 150 °C to 500 °C, preferably from 200 °C to 450 °C, for a period of 1 minute to 24 hours, preferably 2 minutes to 2 hours, more preferably 2-30 minutes, in an atmosphere of air, an inert gas, such as nitrogen or argon, or steam. Reducing agents will generally be present to effect the reduction of a silver compound to metallic silver. For example, a reducing atmosphere, such as a hydrogen containing gas, may be employed, or a reducing agent may be present in one or more of the impregnation liquids, for example oxalate. If desired, the pore impregnation may be carried out in more than one impregnation and drying step. For example, silver may be impregnated in more than one step, and the promoters may be impregnated in one or more separate steps, prior to silver impregnation, after silver impregnation or intermediate to separate silver impregnation steps. The liquid composition is typically a solution, more typically an aqueous solution. The compounds employed in the impregnation may independently be selected from, for example, inorganic and organic salts, hydroxides and complex compounds. They are employed in such a quantity that a catalyst is obtained of the desired composition.

The invention is useful for the oxidation of any olefin which has at least three carbon atoms. Typically the number of carbon atoms is at most ten, more typically at most five. It is most preferred that the number of carbon atoms is three.

Apart from having an olefinic linkage (i.e. a moiety >C=C<), the olefin may comprise another olefinic linkage, or any other kind of unsaturation, for example in the form of an aryl group, for example a phenyl group. Thus, the olefin may be a conjugated or non-conjugated diene or a conjugated or non-conjugated vinyl aromatic compound, for example 1,3-butadiene, 1,7-octadiene, styrene or 1,5-cyclooctadiene.

In preferred embodiments, the olefin comprises a single olefinic linkage and for the remainder it is a saturated hydrocarbon. It may be linear, branched or cyclic. A single alkyl group may be attached to the olefinic linkage, such as in 1-hexene, or two alkyl groups may be attached to the olefinic linkage, such as in 2-methyl-octene-1 or pentene-2. It is also possible that three or four alkyl groups are attached to the olefinic linkage. Two alkyl groups may be linked together with a chemical bond, so that together with the olefinic linkage they form a ring structure, such as in cyclohexene. In these preferred embodiments, a hydrogen atom is attached to the olefinic linkage at the places which are not occupied by an alkyl group. It is particularly preferred that a single alkyl group is attached to the olefinic linkage.

The most preferred olefins having at least 3 carbon atoms are 1-pentene, 1-butene and, in particular, propylene.

The skilled person will appreciate that in accordance with the geometry of the molecules, an olefin may yield a mixture of olefin oxides, for example olefin oxides in more than one isomeric form.

Generally, the process of this invention is carried out as a gas phase process, which is a process wherein gaseous reactants are reacted under the influence of a solid catalyst. Frequently, the reactants and any further components fed to the process are mixed to form a reaction mixture which is subsequently contacted with the catalyst. The ratio of the quantities of the reactants and the further components, if any, and the further reaction conditions are not material to this invention and they may be chosen within wide ranges. As, generally, the mixture contacted with the catalyst is gaseous, the concentrations of the quantities of the reactants and the further components, if any, are specified below as a volume fraction of the mixture in gaseous form.

The concentration of the olefin may suitably be at least 0.1 %v, preferably at least 0.5 %v, and the concentration may suitably be at most 60 %v, preferably at most 50 %v. Preferably, the concentration of the olefin is in the range of from 1 %v to 40 %v. If the olefin is propylene, 1-butene or 1-pentene it is preferred that its concentration is in the range of from 1 %v to 30 %v, in particular from 2 %v to 15 %v.

The concentration of oxygen may suitably be at least 2 %v, typically at least 4 %v, and in practice the concentration is frequently at most 20 %v, preferably at most 15 %v. If the olefin is propylene, 1-butene or 1-pentene it is preferred that the concentration of oxygen is in the range of from 6 %v to 15 %v, preferably from 8 %v to 15 %v. The source of oxygen may be air, but it is preferred that an oxygen containing gas which may be obtained by separation from air is used.

Organic chloride compounds may be added to the reaction mixture as a moderator of the catalyst, improving the selectivity. Examples of such organic chloride compounds are alkyl chlorides and alkenyl chlorides. Methyl chloride, vinyl chloride, 1,2-dichloroethane and in particular ethyl chloride are preferred organic chloride compounds. In the case of propylene, the organic chloride concentration should be at least 20 ppm by volume, more preferably at least 50 ppm by volume, and the concentration may be at most 2000 ppm by volume, in particular at most 1500 ppm by volume, wherein ppm by volume is calculated as the molar quantity of chlorine atoms in the total quantity of the reactant mixture.

The performance of the catalyst may be improved by adding to the reaction mixture a nitrate or nitrite forming compound. A nitrate or nitrite forming compound is a compound which is capable, under the conditions at which it is contacted with the catalyst, of introducing nitrate or nitrite ions on the catalyst. In general, the nitrate or nitrite ions tend to disappear from the catalyst during the process, in which case they need to be replenished. As a consequence, it is preferred to add the nitrate or nitrite forming compound continuously to the reaction mixture, or in a discontinuous mode, at least at the points in time that the need thereto arises. For the initial stage of the process it may be sufficient to add the nitrate or nitrite forming compound or nitrate or nitrite ions to the catalyst at the stage of catalyst preparation. Preferred nitrate or nitrite forming compounds are nitric oxide, nitrogen dioxide and/or dinitrogen tetraoxide. Alternatively, hydrazine, hydroxylamine, ammonia, nitromethane, nitropropane or other nitrogen containing compounds may be used. A mixture of nitrogen oxides is preferably used, which may be designated by the general formula NOₓ, wherein x is a number in the range of from 1 to 2, expressing the molar average atomic ratio of oxygen and nitrogen of the nitrogen oxides in the mixture.

For propylene oxidation, the nitrate or nitrite forming compound may suitably be used at a concentration of at least 10 ppm by volume, typically at least 50 ppm by volume, and the concentration may suitably be at most 500 ppm by volume, in particular at most 300 ppm by volume. If rubidium and/or cesium are present in the catalyst used for propylene oxidation, the nitrate or nitrite forming compound is preferably used at a concentration of at least 10 ppm by volume, in particular at least 20 ppm by volume, and the concentration is typically at most 200 ppm by volume, more typically at most 150 ppm by volume, preferably at most 80 ppm by volume, in particular at most 50 ppm by volume, on the same basis.

Carbon dioxide may or may not be present in the mixture. Carbon dioxide may reduce catalyst activity and selectivity and, thus, the yield of olefin oxide. Carbon dioxide may typically be present at a concentration of at most 35 %v, in particular at most 20 %v.

Furthermore, inert compounds may be present in the mixture, for example nitrogen or argon. In one specific embodiment of the present invention, it is preferred to have methane present in the mixture, as methane may improve the dissipation of the heat of reaction, without adversely effecting the selectivity and the conversion.

The process may preferably be carried out at a temperature of at least 150 °C, in particular at least 200 °C. Preferably the temperature is at most 320 °C, more preferably at most 300 °C. The process may preferably be carried out at a pressure of at least 50 kPa (0.5 barg (i.e. bar gauge)), more preferably at least 100 kPa (1 barg). Preferably, the pressure is at most 10 MPa (100 barg), more preferably at most 5 MPa (50 barg).

In general, it is preferred to operate at a high oxygen concentration. However, in actual practice in order to remain outside the flammability limits of the mixture of reactants and any further components present therein, the concentration of oxygen has to be lowered as the concentration of the olefin is increased. The actual safe operating conditions depends along with the gas composition, also on individual plant conditions, such as temperature and pressure.

When operating the process as a gas phase process using a packed bed reactor, the GHSV may preferably be at least 1000 Nl/(l.h), in particular at least 2000 Nl/(l.h). The GHSV may preferably be at most 15000 Nl/(l.h), in particular at most 10000 Nl/(l.h). The term "GHSV" stands for the Gas Hourly Space Velocity, which is the volumetric flow rate of the feed gas, which is herein defined at normal conditions (i.e. 0 °C and 1 bar absolute), divided by the volume of the catalyst bed.

The product of the process of this invention, the olefin oxide, may or may not be converted into the corresponding 1,2-diol or a 1,2-diol ether. The conversion into the 1,2-diol or 1,2-diol ethers may comprise, for example, reacting the olefin oxide with water, suitably using an acidic or a basic catalyst. For example, for making predominantly the 1,2-diol rather than the 1,2-diol ethers, the olefin oxide may be reacted with a ten fold molar excess of water, in a liquid phase reaction in the presence of an acid catalyst, e.g. 0.5-1.0 %w sulfuric acid, based on the total reaction mixture, at 50-70 °C at 100 kPa (1 bar absolute), or in a gas phase reaction at 130-240 °C and 2-4 MPa (20-40 bar), preferably in the absence of a catalyst. If the proportion of water is lowered, the proportion of 1,2-diol ethers in the reaction mixture is increased. The 1,2-diol ethers thus produced may be the di-ether, tri-ether, tetra-ether and subsequent ethers. Alternative 1,2-diol ethers may be prepared by converting the olefin oxide with an alcohol, in particular a primary alcohol, such as methanol or ethanol, by replacing at least a portion of the water by the alcohol.

The 1,2-diols and 1,2-diol ethers may be used in a large variety of industrial applications, for example in the fields of food, beverages, tobacco, cosmetics, thermoplastic polymers, curable resin systems, detergents, heat transfer systems, etc.

Unless specified otherwise, the low-molecular weight organic compounds mentioned herein have suitably at most 20 carbon atoms, typically at most 10 carbon atoms, more typically at most 6 carbon atoms. Organic compounds are deemed to be compounds which comprise carbon atoms and hydrogen atoms in their molecules. As defined herein, ranges for numbers of carbon atoms (i.e. carbon number) include the numbers specified for the limits of the ranges. Number of carbon atoms as defined herein include the carbon atoms along the carbon backbones, as well as branching carbon atoms, if any.

The invention will now be illustrated by the following non-limiting examples.

### EXAMPLE A

### Preparation of Silver-Amine Oxalate Stock Solution

A silver-amine-oxalate stock solution was prepared by the following procedure:
415 g of reagent-grade sodium hydroxide were dissolved in 2340 ml de-ionized water and the temperature was adjusted to 50 °C. 1699 g high purity "Spectropure" silver nitrate was dissolved in 2100 ml de-ionized water and the temperature was adjusted to 50 °C. The sodium hydroxide solution was added slowly to the silver nitrate solution, with stirring, while maintaining a solution temperature of 50 °C. This mixture was stirred for 15 minutes, then the temperature was lowered to 40 °C. Water was removed from the precipitate created in the mixing step and the conductivity of the water, which contained sodium and nitrate ions, was measured. An amount of fresh deionized water equal to the amount removed was added back to the silver solution. The solution was stirred for 15 minutes at 40 °C. The process was repeated until the conductivity of the water removed was less than 90 µmho/cm. 1500 ml fresh deionized water was then added.
630 g of high-purity oxalic acid dihydrate were added in approximately 100 g increments. The temperature was maintained at 40 °C and the pH was kept at a level above 7.8. Water was removed from this mixture to leave a highly concentrated silver-containing slurry. The silver oxalate slurry was cooled to 30 °C. Then 699 g of 92 %w ethylenediamine (8% de-ionized water) was added to the slurry while maintaining a temperature no greater than 30 °C. The resulting solution contained approximately 27-33 %w silver.

### EXAMPLE B

A calcium carbonate carrier useful in the catalysts of the invention was prepared as follows: 100 parts by weight (pbw) calcium carbonate were mixed with 2 pbw of an organic extrusion aid such as starch. 45 pbw silver solution prepared as shown in Example A were added and the resulting composition was mixed until homogeneous and extrudable. The resulting paste was forced through a 3 mm die. The resulting extrudate was dried overnight at 110 °C and then fired as follows: 5 hour ramp to 500 °C held for 5 hours.

### Examples 1-18 (Examples 1-16 for comparison; Examples 17 and 18 according to the invention)

Catalysts were prepared by pore impregnating a molded porous carrier which was an alpha-alumina, obtained from Norton Chemical Process Products Corporation, which had a BET surface area of 0.8 m²/g and an apparent porosity, or water absorption, of 0.4 ml/g. The impregnation was effected in a single impregnation step using solutions prepared from silver nitrate, and nitrates or hydroxides of alkali metals, applying the method known from US-A-4833261, Illustrative Embodiment 1. The impregnated alpha-alumina was dried, and heated at 250 °C for 5 minutes. The moldings were crushed and sieved to 12-20 mesh. The content of silver was 14 %w, based on the weight of the catalyst composition, the content of the alkali metals was as indicated in Table I.

Samples (5 g) of the 12-20 mesh particles so obtained were loaded into a micro-reactor for testing the catalyst performance in the oxidation of propylene. The test conditions were as follows. The feed gas composition was 8 %v oxygen, 5 %v propylene, 100 ppmw NOₓ, 150 ppmw ethyl chloride, based on the total volume or weight, as appropriate, of the gas. The remainder of the feed gas was nitrogen. The gas was fed at a rate of 9 Nl/h. The temperature was as indicated in Table I, the pressure was 350 kPa (3.5 barg).

The results on the catalyst performance, measured as the selectivity and the work rate at the point in time that the selectivity had stabilized, are shown in Table I. The selectivity is expressed as the %mole of propylene oxide produced, relative to the propylene consumed. The work rate is the rate of propylene oxide production per unit weight of catalyst (kg/(kg.h)).

**Table I**

| Ex. | Alkali metal (µmoles/g) | | | | | Temp. (°C) | Selectivity (%mole) | Work rate (kg/ (kg.h)) |
|---|---|---|---|---|---|---|---|---|
| | Li | Na | K | Rb | Cs | | | |
| 1 | - | - | - | - | - | 280 | 14 | 4 |
| 2 | - | - | - | - | - | 250 | 13 | 4 |
| 3 | 40 | 32 | - | - | - | 250 | 11 | 7 |
| 4 | - | 50 | - | - | - | 280 | 11 | 9 |
| 5 | - | - | 9 | - | - | 280 | 11 | 10 |
| 6 | - | - | 18 | - | - | 280 | 44 | 21 |
| 7 | - | - | 56 | - | - | 280 | 43 | 20 |
| 8 | - | - | 60 | - | - | 250 | 43 | 14 |
| 9 | - | - | 280 | - | - | 280 | 42 | 18 |
| 10 | - | - | - | 6 | - | 280 | 11 | 17 |
| 11 | - | - | - | 10 | - | 280 | 0 | 0 |
| 12 | - | - | - | 50 | - | 280 | 0 | 0 |
| 13 | - | - | - | - | 1 | 280 | 6 | 1 |
| 14 | - | - | - | - | 2 | 260 | 6 | 2 |
| 15 | - | - | - | - | 5 | 250 | 7 | 2 |
| 16 | - | - | 18 | - | 13 | 280 | 42 | 18 |
| 17 *) | 40 | - | 56 | - | - | 250 | 48 | 15 |
| 18 *) | - | 32 | 32 | - | - | 280 | 46 | 20 |

| | | | | | | | | |
|---|---|---|---|---|---|---|---|---|
| *) According to the invention | | | | | | | | |

The results in Table I show that, except for potassium, all alkali metals have a negative effect on the selectivity and/or work rate of the catalyst, when compared to the case where no alkali metal is added (compare Examples 3, 4 and 10-15 with Examples 1 and 2). The positive effect of potassium is advantageous for all concentrations tested (compare Example 1 with Examples 5-9). However, beyond a concentration of 18 µmole/g of potassium no further improvement in catalyst performance is seen (compare Examples 7 and 9 with Example 6). The addition of 13 µmole/g of cesium to 18 µmole/g of potassium did not improve the catalyst performance (compare Example 16 with Example 6).

In Example 17, according to the invention, it can be seen that an improvement in the catalyst performance can be achieved by the addition of lithium and potassium, even at the concentration level of potassium where no further improvement is seen by adding more potassium (compare Example 17 with Example 7). In Example 18 (according to the invention), the same effects can be seen for the addition of sodium and potassium (compare Example 18 with Examples 6 and 7).

### Examples 19-22 (Examples 19 and 20 for comparison; Examples 21 and 22 according to the invention)

The procedures as described for Examples 1-18 were repeated with the following differences:
- the alpha-alumina had a BET surface area of 2.0 m²/g, instead of 0.8 m²/g, and a water absorption of 0.4 ml/g;
- before impregnating, the carrier was washed by immersing the carrier in three portions of boiling de-ionised water (300 g per 100 g carrier) in each portion for 15 minutes, followed by drying in a ventilated oven at 150 °C for 18 hours;
- the quantity of silver was 24 %w, instead of 12 %w, relative to the weight of the catalyst composition;
- half of the silver was added to the carrier by a separate impregnation step, preceding the impregnation of the remainder of the silver and the alkali metal;
- samples of 15 g were loaded into a micro-reactor, instead of samples of 5 g; and
- the feed gas contained 12 %v of oxygen and 8 %v of propylene, instead of 8 %v and 5 %v, respectively.

Particulars of the test conditions and the results on the catalyst performance are given in Table II.

**Table II**

| Ex. | Alkali metal (µmole/g) | | | | | Temp. (°C) | Selectivity (%mole) | Work rate (kg/ (kg.h)) |
|---|---|---|---|---|---|---|---|---|
| | Li | Na | K | Rb | Cs | | | |
| 19 | - | - | 56 | - | - | 250 | 44 | 18 |
| 20 | - | - | 250 - | | - | 250 | 45 | 15 |
| 21 *) | 40 | 60 | 60 | - | - | 250 | 55 | 15 |
| 22 *) | 160 | 100 | 100 - | | - | 250 | 57 | 9 |

| | | | | | | | | |
|---|---|---|---|---|---|---|---|---|
| *) According to the invention | | | | | | | | |

- before impregnating, the carrier was washed by immersing the carrier in three portions of boiling de-ionised water (300 g per 100 g carrier) in each portion for 15 minutes, followed by drying in a ventilated In Examples 21 and 22, according to the invention, it can be seen that an improvement in the catalyst performance can be achieved by the addition of lithium and sodium to potassium, even at the concentration level of potassium where no further improvement is seen by adding more potassium (compare with Examples 19 and 20).

### Examples 23-28 (according to the invention)

The procedures as described for Examples 1-18 were repeated with the following differences:
- the alpha-alumina had a BET surface area of 2.0 m²/g, instead of 0.8 m²/g, and an apparent porosity of 0.4 ml/g, measured by water absorption;
   oven at 150 °C for 18 hours;
- the quantity of silver was 23 %w, instead of 12 %w, relative to the weight of the catalyst composition;
- half of the silver was added to the carrier by a separate impregnation step, preceding the impregnation of the remainder of the silver and the alkali metal;
- samples of 15 g were loaded into a micro-reactor, instead of samples of 5 g; and
- the feed gas contained 12 %v of oxygen and 8 %v of propylene, instead of 8 %v and 5 %v, respectively.

Particulars of the test conditions and the results on the catalyst performance are given in Table III.

**Table III**

| Ex. | Alkali metal (µmole/g) | | | | | Temp. (°C) | Selectivity (%mole) | Work rate (kg/ (kg.h)) |
|---|---|---|---|---|---|---|---|---|
| | Li | Na | K | Rb | Cs | | | |
| 23 *) | - | 40 | 40 | - | - | 230 | 56 | 7 |
| 24 *) | - | 40 | 40 - | | 3 | 230 | 59 | 12 |
| 25 *) | - | 40 | 40 | 5 | - | 230 | 55 | 12 |
| 26 *) | 40 | - | 40 | - | - | 230 | 33 | 9 |
| 27 *) | 40 | - | 40 | - | 3 | 230 | 44 | 13 |
| 28 *) | 40 | 40 | 40 | - | 3 | 230 | 62 | 5 |

| | | | | | | | | |
|---|---|---|---|---|---|---|---|---|
| *) According to the invention | | | | | | | | |

In Examples 24, 25, 27 and 28, according to the invention, it can be seen that a further improvement in the catalyst performance can be achieved by the addition of rubidium or cesium to potassium, lithium and sodium (compare with Examples 23 and 26, according to the invention). The improvement may in the selectivity and/or in the work rate.

### Examples 29 and 30 (Example 29 for comparison; Example 30 according to the invention)

The procedures as described for Examples 1-18 were repeated with the difference that the performance of the catalyst was followed over a 10-day period.

Particulars of the test conditions and the results are given in Table IV.

**Table IV**

| Ex. | Alkali metal (µmole/g) | | | | | Temp. (°C) | Work rate (kg/(kg.h)) | |
|---|---|---|---|---|---|---|---|---|
| | Li | Na | K | Rb | Cs | | After 1 day | After 10 days |
| 29 | - | - | 60 | - | - | 280 | 25 | 8 |
| 30 *) | 40 | 32 | 32 | - | - | 280 | 26 | 18 |

| | | | | | | | | |
|---|---|---|---|---|---|---|---|---|
| *) According to the invention | | | | | | | | |

By comparing Example 30, according to the invention, with Example 29 it can be seen that over an extended period of time, the performance of a catalyst according to the invention is more stable than a comparative catalyst.

### Examples 31-37 (Examples 31-34 for comparison;

### Examples 35-37 according to the invention)

The procedures as described for Examples 1-18 were repeated with the following differences:
- the porous carrier was a calcium carbonate prepared as in Example C;
- the quantity of silver added to this carrier was 26 %w, instead of 12 %w, relative to the weight of the catalyst composition;
- half of the silver was added to the carrier by a separate impregnation step, preceding the impregnation of the remainder of the silver and the alkali metal;
- catalyst samples of various weights were loaded into a micro-reactor, instead of samples of 5 g; and
- the feed gas contained 12 %v of oxygen and 8 %v of propylene, instead of 8 %v and 5 %v, respectively, unless mentioned otherwise.

Particulars of the test conditions and the results on the catalyst performance, measured as the selectivity and the work rate at the point in time after 2 days testing, are given in Table V.

**Table V**

| Ex. | Catalyst (g) | Alkali metal (µmole/g) | | | | | Temp. (°C) | Selectivity (%mole) | Work rate (kg/ (kg.h)) |
|---|---|---|---|---|---|---|---|---|---|
| | | Li | Na | K | Rb | Cs | | | |
| 31 | 15 | - | - | 56 | - | - | 250 | < 10 | < 3 |
| 32 *) | 5 | - | - | 250 | - | - | 250 | 42 | 20 |
| 33 *) | 5 | - | - | 500 | - | - | 250 | 42 | 25 |
| 34 | 18 | - | - | 1000 | - | - | 250 | 43 | 16 |
| 35 **) | 15 | 50 | 50 | 500 | - | - | 250 | 52 | 15 |
| 36 **) | 15 | 50 | 200 | 500 | - | - | 250 | 54 | 9 |
| 37 **) | 15 | 50 | 500 | 500 | - | - | 250 | 52 | 10 |

| | | | | | | | | | |
|---|---|---|---|---|---|---|---|---|---|
| *) The feed gas contained 8 %v of oxygen and 5 %v of propylene **) According to the invention | | | | | | | | | |

In Examples 35-37, according to the invention, it can be seen that an improvement in the catalyst performance can be achieved by the addition of lithium and sodium to potassium, even at the concentration level of potassium where no further improvement is seen by adding more potassium (compare with Examples 31-34).

The instant application shows a detailed description of particular embodiments of the invention as described above. It is understood that all equivalent features are intended to be included within the claimed contents of this invention.

## Claims

1. A process for preparing a catalyst composition comprising:
depositing silver on a carrier; and
depositing an alkali metal promoter on the carrier,
which alkali metal promoter comprises potassium in a quantity of at least 10 µmole/g, relative to the weight of the catalyst composition, and lithium in a quantity of at least 1 µmole/g, relative to the weight of the catalyst composition, wherein the alkali metal promoter is deposited simultaneously with, prior to, or after depositing silver on the carrier.

2. The process of claim 1, wherein potassium is deposited in a quantity of at least 32 µmole/g, relative to the weight of the catalyst composition.

3. The process of claim 1, wherein lithium is deposited in a quantity of at least 5 µmole/g, relative to the weight of the catalyst composition.

4. The process of claim 1, wherein the process further comprises depositing sodium in a quantity of at least 5 µmole/g, relative to the weight of the catalyst composition, simultaneously with, prior to, or after depositing silver on the carrier.

5. The process of claim 4, wherein lithium and sodium are deposited in a sodium/lithium molar ratio in the range of from 0.01 to 100.

6. The process of claim 4, wherein lithium and sodium are deposited in a sodium/lithium molar ratio in the range of from 0.1 to 10.

7. The process of claim 1, wherein the carrier comprises an α-alumina having a BET surface area of 0.1 m²/g to 25 m²/g, and an apparent porosity of from 0.1 ml/g to 1.2 ml/g, measured by water absorption.

8. The process of claim 1, wherein the carrier comprises a silver bonded calcium carbonate having a crush strength of at least 22 N.

9. The process of claim 1, wherein the carrier comprises a silver bonded calcium carbonate wherein the weight ratio of silver to calcium carbonate is from 1:5 to 1:100.

10. The process of claim 1, wherein the carrier comprises a silver bonded calcium carbonate having a specific surface area of from 1 m²/g to 20 m²/g.

11. The process of claim 1, wherein the carrier comprises a silver bonded calcium carbonate having an apparent porosity of from 0.05 ml/g to 2 ml/g.

12. The process of claim 1, wherein the carrier comprises at least 95% w α-alumina.

13. A process for preparing a catalyst composition comprising:
depositing silver on a carrier; and
depositing an alkali metal promoter on the carrier,
which alkali metal promoter comprises potassium in a quantity of at least 10 µmole/g, relative to the weight of the catalyst composition, and sodium in a quantity of at least 5 µmole/g, relative to the weight of the catalyst composition, wherein the alkali metal promoter is deposited simultaneously with, prior to, or after depositing silver on the carrier.

14. The process of claim 13, wherein sodium is deposited in a quantity of at least 10 µmole/g, relative to the weight of the catalyst composition.

15. The process of claim 13, wherein sodium is deposited in a quantity of at least 32 µmole/g, relative to the weight of the catalyst composition.

16. The process of claim 15, wherein potassium is deposited in a quantity of at least 32 µmole/g, relative to the weight of the catalyst composition.

17. A process for preparing an olefin oxide by reacting an olefin having at least 3 carbon atoms with oxygen in the presence of a catalyst prepared by
depositing silver on a carrier; and
depositing an alkali metal promoter on the carrier, which alkali metal promoter comprises potassium in a quantity of at least 10 µmole/g, relative to the weight of the catalyst composition, and lithium in a quantity of at least 1 µmole/g, relative to the weight of the catalyst composition, wherein the alkali metal promoter is deposited simultaneously with, prior to, or after depositing silver on the carrier.

18. The process of claim 17 which is further conducted in the presence of a nitrate or nitrite forming compound.

19. The process of claim 17, wherein the olefin comprises propylene.

20. A process for producing a 1,2-diol or a 1,2-diol ether comprising:
obtaining an olefin oxide by reacting an olefin having at least 3 carbon atoms with oxygen in the presence of a catalyst prepared by
depositing silver on a carrier; and
depositing an alkali metal promoter on the carrier, which alkali metal promoter comprises potassium in a quantity of at least 10 µmole/g, relative to the weight of the catalyst composition, and lithium in a quantity of at least 1 µmole/g, relative to the weight of the catalyst composition, wherein the alkali metal promoter is deposited simultaneously with, prior to, or after depositing silver on the carrier; and
converting the olefin oxide into the 1,2-diol or the 1,2-diol ether.
